# EUROPEAN PATENT APPLICATION

(11) **EP 2 127 673 A1**
(43) Date of publication of application: **02.12.2009**
(21) Application number: 08720365.9
(22) Date of filing: 07.03.2008
(51) Int. Cl.: A61K 45/00, A61K 31/231, A61P 17/12, A61P 29/00

(54) **PHARMACEUTICAL AGENT FOR PREVENTION AND TREATMENT OF SKIN DISEASE INDUCED BY ACCELERATED KERATINIZATION**

(30) Priority: 09.03.2007 JP 2007059519
(71) Applicant: Kyoto University, Kyoto-shi Kyoto 606-8501 (JP)
(72) Inventor: TAKAHASHI, Kenzo, Kyoto-shi Kyoto 606-8501 (JP)
(74) Representative: Polypatent
(86) International application number: PCT/JP2008/000479
(87) International publication number: WO 2008/111296

(57) **Abstract**

A medicament for prophylactic and/or therapeutic treatment of a dermatosis resulting from excessively advanced keratinization, such as non-hereditary inflammatory keratosis and hereditary congenital keratosis, which comprises a substance selected from the group consisting of a cannabinoid agonist and a vanilloid agonist as an active ingredient.

## Description

### Technical Field

The present invention relates to a medicament for prophylactic and/or therapeutic treatment of a dermatosis. More specifically, the present invention relates to a medicament effective for prophylactic and/or therapeutic treatment of a dermatosis resulting from excessively advanced keratinization, such as non-hereditary inflammatory keratosis and hereditary congenital keratosis.

### Background Art

Darie's disease is a dominantly inherited dermatosis, in which expression amount of ATP2A2 calcium pump (i.e., a pump existing in endoplasmic reticulum and responsible for calcium ion transport from cytoplasm to endoplasmic reticulum) in epidermal keratinocytes decreases to about half of that observed in healthy persons due to genetic mutation in Serca2 gene. As a result, abnormality of calcium metabolism in the epidermal keratinocytes is affected and normal keratinization process is disturbed, which causes cutaneous symptoms such as individual keratinization, dyskeratosis, and acantholysis. In this disease, one normal allele of the Serca2 gene is kept remained, and the ATP2A2 protein normally expressed from that gene is present at an amount nearly half of that in healthy persons. However, the aforementioned amount is sufficient as a protein amount to perform the physiological calcium metabolism, which triggers the onset of the disease. This critical mechanism is referred to as haplo-insufficiency, and is known as pathological mechanism of several human dominantly inherited diseases other than Darie's disease. For example, also in Hailey-Hailey disease, it is known that the calcium pump existing in the Golgi apparatus becomes insufficient due to the haplo-insufficiency of Spca1 gene, resulting in inducing abnormality of calcium metabolism in keratinocytes.

Darie's disease is characterized in sudden onset around or after the age of adolescence, and by the aggravation in summer but remission in winter season, although the disease is a dominantly inherited dermatosis. Keratotic erosions and skin ulcers are widely developed in seborrheic area in face, thorax and the like, and fetor often accompanies- For therapeutic treatment of Darie's disease, glucocorticoid steroid agents for external use, retinoic acid for oral administration, active vitamin D3 preparations for external use and the like have been currently used. However, these drugs are therapeutic agents mainly for improving secondary changes of skin, such as erosion, inflammation, and infection, and are not medicaments that successfully achieve radical treatment of Darie's disease. Under the circumstances, it is strongly desired to provide a medicament that can achieve curative treatment of Darie's disease.

Cannabinoid is a general term that encompasses a large number of physiologically active substances including marihuana, and the CB1 receptor and CB2 receptor are known as cannabinoid receptors. It is known that the CB1 receptor is expressed in central and peripheral nerves, and involved in central actions such as memory and learning and peripheral actions such as inflammation response and pain relief, and the CB2 receptor is expressed in immunocytes, mast cells, and epidermal keratinocytes, and involved in peripheral actions such as inflammation response and pain relief. As cannabinoid agonists, for example, CB2 receptor antagonists such as indomethacin morphonylamide and palmidrol, as well as non-selective agonists such as anandamide and tetrahydrocannabinoid are known.

With regard to the cannabinoid agonists, Japanese Patent Unexamined Publication (KOKAI) No. 2000-256323 discloses cannabinoid agonists having an immuno-regulatory action, anti-inflammatory action, and anti-allergic action, and Japanese Patent Unexamined Publication based on International Patent Application (KOHYO) No. 11-500411 discloses that cannabinoids are useful for therapeutic treatment of diseases relating to abnormality of peripheral cannabinoid receptors, and specifically, discloses that they can be used for diseases accompanied by dysalgesia, multiple sclerosis, diseases accompanied by abnormality of intraocular pressure, and chronic degenerative disorders such as chronic respiratory disorder, senile dementia, and Alzheimer's disease. Japanese Patent Unexamined Publication No. 5-345722 discloses that N-palmitoyl-ethanolamide (palmidrol) as a cannabinoid agonist is useful as a therapeutic agent for autoimmune diseases such as atopic dermatitis and dermatomyositis.
Furthermore, Japanese Patent Unexamined Publication No. 2003-201250 discloses that cannabinoid agonists have superior anti-pruritus action, and are useful as therapeutic agents of pruritus such as eye pruritus, skin pruritus, and systemic pruritus.

A typical example of vanilloid is capsaicin, which is a red pepper ingredient. A vanilloid receptor is widely expressed in sensory nerves and epidermal tissues that are directly contacted with foreign stimulus, which is a cationic channel type receptor involved in noxious stimulus reception. As vanilloid receptors, the TRPV (transient receptor potential vanilloid) superfamily is known, and six kinds of subtypes have been reported so far. Among them, those expressed in the skin are five kinds, i.e., TRPV1, TRPV3, TRPV4, TRPV5 and TRPV6- It has been revealed that TRPV1 relates to pain stimulus, and TRPV3 and TRPV4 are activated by thermal stimuli, mechanical stress, osmotic pressure, and the like.

With regard to the vanilloid agonists, a method of treating pain, inflammatory hyperalgesia, urocystitis, prostatic hyperplasia, herpes, dermatitis, pruritus, psoriasis, skin cancer, wrinkles and the like by allowing a vanilloid agonist to act on the skin (WO2004/091521), and therapeutic use of capsaicin for ulcer, gangrene and the like developed in extremities in patients with pain of extremities or dysesthesia resulting from diabetes (Japanese Patent Unexamined Publication No. 2006-160644) are known.

However, it is not known so far that cannabinoid agonists and vanilloid agonists are effective for a dermatosis resulting from abnormal calcium metabolism in keratinocytes such as Darie's disease. WO2003/075908 describes that adapalene (trade name: Differin), which is a kind of retinoid, is effective for psoriasis, ichthyosis, Darie's disease, keratosis pilaris, inflammatory dermatosis, and the like. However, the patent document does not specifically discloses any experimental results demonstrating effectiveness thereof for Darie's disease.
Patent document 1: Japanese Patent Unexamined Publication No. 2000-256323
Patent document 2: Japanese Patent Unexamined Publication based on International Patent Application No. 11-500411
Patent document 3: Japanese Patent Unexamined Publication No. 5-345722
Patent document 4: Japanese Patent Unexamined Publication No. 2003-201250
Patent document 5: WO2004/091521
Patent document 6: Japanese Patent Unexamined Publication No. 2006-160644

### Disclosure of the Invention

### Object to be Achieved by the Invention

An object of the present invention is to provide a medicament effective for prophylactic and/or therapeutic treatment of a dermatosis resulting from excessively advanced keratinization, for example, non-hereditary inflammatory keratosis and hereditary congenital keratoderma. More specifically, the object of the present invention is to provide a medicament effective for prophylactic and/or therapeutic treatment of dermatoses resulting from excessively advanced keratinization including, for example, Darie's disease and Hailey-Hailey disease, which are congenital dermatoses directly resulting from abnormal calcium metabolism in epidermal keratinocytes, and dermatoses resulting from excessively advanced keratinization other than Darie's disease and Hailey-Hailey disease including congenital keratosis, psoriasis vulgaris, and the like.

### Means for Achieving the Object

The inventors of the present invention conducted various researches to achieve the aforementioned object, and as a result, they found that both the CB1 receptor and CB2 receptor were expressed in the epidermal keratinocytes. They also found that a cannabinoid agonist or a vanilloid agonist stimulated the expression of the Serca2 gene in the epidermal keratinocytes to increase an expression amount of the ATP2A2 calcium pump in the epidermal basal layer, and the agonist was useful for prophylactic and/or therapeutic treatment of a dermatosis resulting from abnormal calcium metabolism in the epidermal keratinocytes such as Darie's disease due to the action mentioned above. The present invention was accomplished on the basis of the aforementioned findings.

The present invention thus provides a medicament for prophylactic and/or therapeutic treatment of a dermatosis resulting from excessively advanced keratinization, which comprises a substance selected from the group consisting of a cannabinoid agonist and a vanilloid agonist as an active ingredient.
According to preferred embodiments of the present invention, there are provided the aforementioned medicament, wherein the dermatosis resulting from the excessively advanced keratinization is inflammatory keratosis (non-hereditary) or congenital keratoderma (hereditary); the aforementioned medicament, wherein the dermatosis resulting from the excessively advanced keratinization is a congenital dermatosis resulting from abnormal calcium metabolism in epidermal keratinocytes; the aforementioned medicament, wherein the congenital dermatosis resulting from the abnormal calcium metabolism in epidermal keratinocytes is Darie's disease or Hailey-Hailey disease; and the aforementioned medicament, which comprises a vanilloid agonist as an active ingredient.

From other aspects of the present invention, there are provided use of a substance selected from the group consisting of a cannabinoid agonist and a vanilloid agonist for manufacture of the aforementioned medicament; and a method for prophylactic and/or therapeutic treatment of a dermatosis resulting from excessively advanced keratinization, for example, a congenital dermatosis resulting from abnormal calcium metabolism in epidermal keratinocytes, which comprises the step of administering a substance selected from the group consisting of a cannabinoid agonist and a vanilloid agonist.

### Effect of the Invention

The medicament of the present invention promotes the expression of the ATP2A2 calcium pump in the endoplasmic reticulum and reduces intracellular calcium concentration, and thereby abnormal excessively advanced keratinization can be controlled to advance normal keratinization. Therefore, the medicament of the present invention exhibits extremely high effectiveness for treatment of a dermatosis resulting from excessively advanced keratinization, for example, inflammatory keratosis (non-hereditary) or congenital keratoderma (hereditary), preferably Darie's disease and Hailey-Hailey disease, which are congenital dermatoses resulting from abnormal calcium metabolism in epidermal keratinocytes. By using the medicament of the present invention, radical treatment of intractable congenital keratoderma including Darie's disease and Hailey-Hailey disease, to which only symptomatic therapies has been applicable as therapeutic means, is achievable, and accordingly, the medicament of the present invention is extremely useful.

### Brief Description of the Drawings

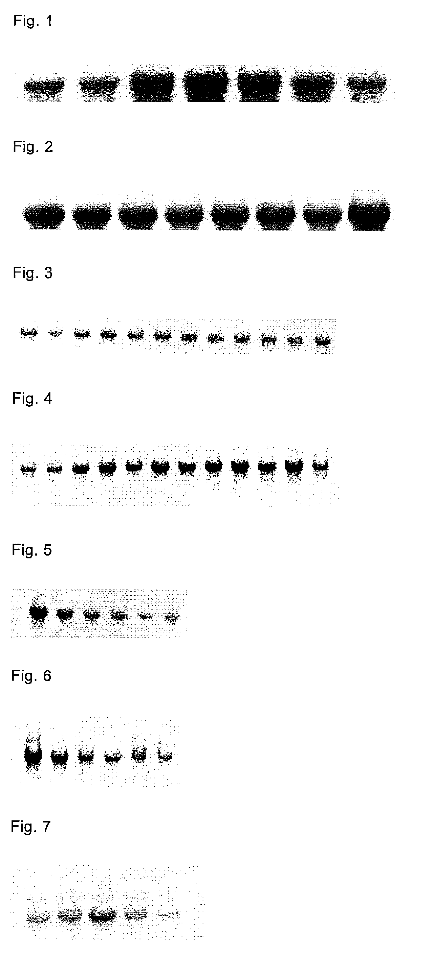

[Fig. 1] This figure depicting the results of expression of Serca2 gene observed by Northern blotting using RNAs extracted from epidermal keratinocytes after addition of WiN55,212-2. The results after 0, 3, 6, 12, 18, 24, and 48 hours are shown from the left.
[Fig. 2] This figure depicts the results of expression of Serca2 gene observed by Northern blotting using RNAs extracted from epidermal keratinocytes 6 hours after the addition of various concentrations of WiN65,212-2 or DMSO as a control. The results obtained at concentrations of 10⁻⁵, 10⁻⁶, 10⁻⁷, 3 x 10⁻⁸, 10⁻⁸, 3 x 10⁻⁹, and 10⁻⁹ mol are shown from the right, and the leftmost is the result obtained by the control.
[Fig. 3] This figure depicts the results of expression of Serca2 gene observed by Northern blotting using RNAs extracted from epidermal keratinocytes 6 hours after the addition of various kinds of vanilloid agonists. The results obtained by palmitoyldopamine, oleoyldopamine, linoleoyldopamine, γ-linolenoyldopamine, eicosa-11Z,14Z-dienoyldopamine, dihomo-γ-linolenoyldopamine, arachidonoyldopamine, eicosapentaenoyldopamine, docosatetra-7Z,10Z,13Z,16Z-enoyldopamine, docosahexaenoyldopamine each at 10⁻⁵ mol, and DMSO (control) are shown from the left.
[Fig. 4] This figure depicts the results of expression of Serca2 gene observed by Northern blotting using RNAs extracted from epidermal keratinocytes 6 hours after the addition of various kinds of vanilloid agonists. The results obtained by palmitoyldopamine, oleoyldopamine, linoleoyldopamine, γ-linolenoyldopamine, eicosa-11Z,14Z-dienoyldopamine, dihomo-γ-linolenoyldopamine, arachidonoyldopamine, eicosapentaenoyldopamine, docosatetra-7Z,10Z,13Z,16Z-enoyldopamine, docosahexaenoyldopamine each at 10⁻⁷ mol, and DMSO (control) are shown from the left.
[Fig. 5] This figure depicts the results of expression of Serca2 gene observed by Northern blotting using RNAs extracted from epidermal keratinocytes 6 hours after the addition of dihomo-γ-linolenoyldopamine as a vanilloid agonist, or DMSO (control). The results obtained by dihomo-γ-linolenoyldopamine at concentrations of 10⁻⁵, 10⁻⁶, 10⁻⁷, 10⁻⁸, and 10⁻⁹ mol and DMSO (rightmost result) are shown from the left.
[Fig. 6] This figure depicts the results of expression of Serca2 gene observed by Northern blotting using RNAs extracted from epidermal keratinocytes 6 hours after the addition of eicosa-11Z,14Z-dienoyldopamine as a vanilloid agonist, or DMSO (control). The results obtained by eicosa-11Z,14Z-dienoyldopamine at concentrations of 10⁻⁵, 10⁻⁶, 10⁻⁷, 10⁻⁸, and 10⁻⁹ mol and DMSO (rightmost result) are shown from the left.
[Fig. 7] This figure depicts the results of expression of Serca2 gene observed by Northern blotting using RNAs extracted from epidermal keratinocytes 6 hours after the addition of a vanilloid agonist or a vanilloid antagonist. The results obtained by DMSO (control), optimum concentrations of dihomo-γ-linolenoyldopamine, 2-aminoethoxydiphenyl borate (2-APB), 4-α-phorbol 12,13-didecanoate (4α-PDD), and dihomo-γ-linolenoyldopamine + DPTHF (vanilloid inhibitor) are shown from the left.

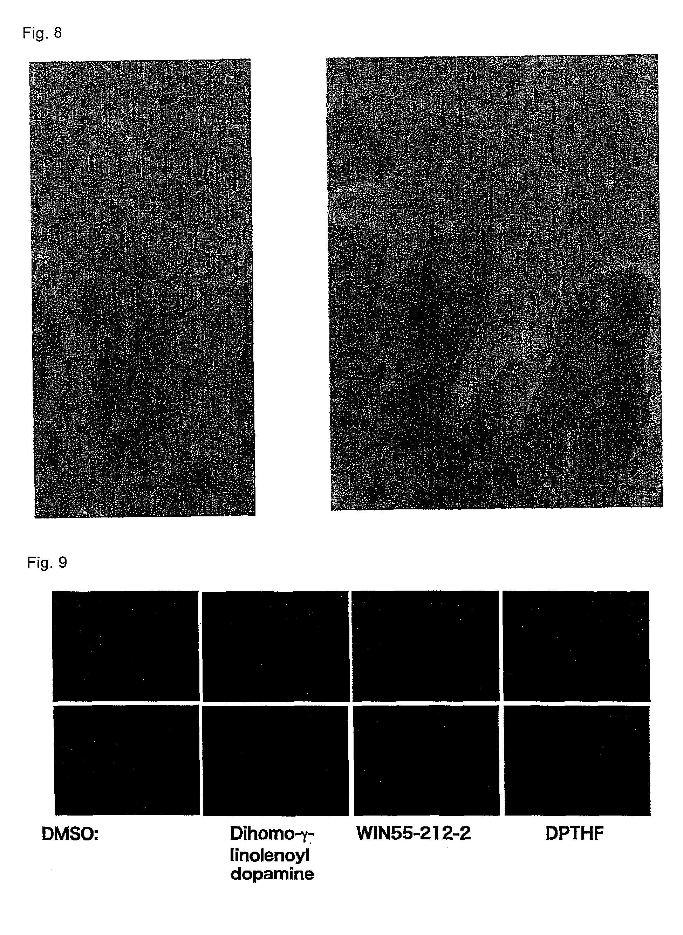

[Fig. 8] This figure depicts the results of identification of localization of the ATP2A protein by immunostaining, a causative protein of Darie's disease. Human skin samples were kept under organ culture condition and added with an optimum concentration of dihomo-γ-linolenoyldopamine or DMSO (control), then fixed after 24 hours. The right figure shows the result of a sagital section added with dihomo-γ-linolenoyldopamine, and the left figure shows the result obtained by DMSO.
[Fig. 9] This figure depicts the results of identification of localization of the ATP2A protein by immunostaining. Human skin samples of healthy subjects were transplanted to nude mice, and after acceptance of graft, each optimum concentration of dihomo-γ-linolenoyldopamine, WIN55,212-2, DPTHF as a vanilloid antagonist, or DMSO (control) was applied every day for 5 days. The ATP2A2 protein is shown in green color in the upper row, and localization of keratin protein representing epidermis are shown in red color in the lower row.

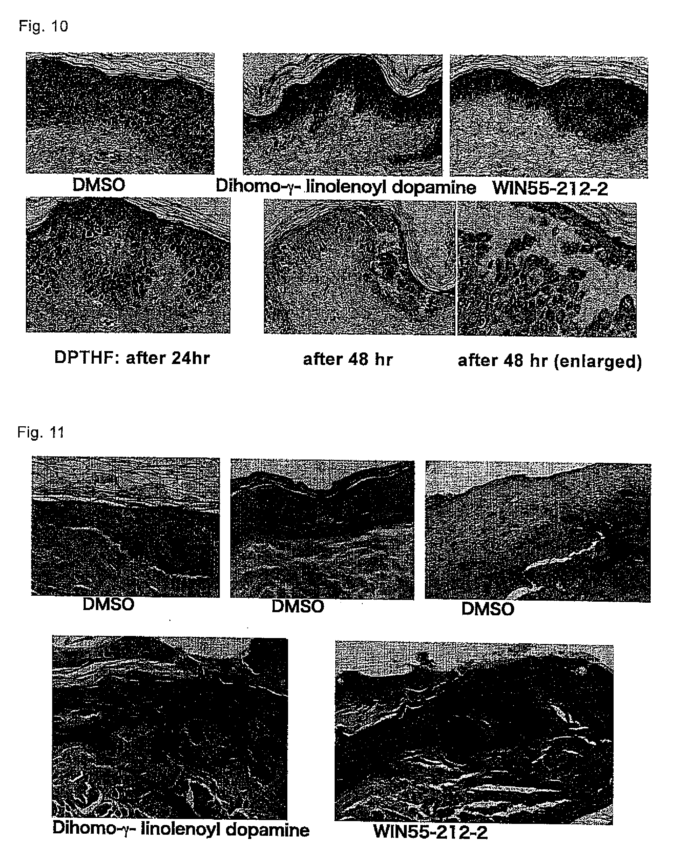

[Fig. 10] This figure depicts the results of the pathological change observed by HE staining, which was carried out by adding each optimum concentration of dihomo-γ-linolenoyldopamine, WIN55,212-2, DPTHF as a vanilloid antagonist, or DMSO (control) to human skin samples of healthy subjects under organ culture condition and fixing the skin after 24 to 48 hours.
[Fig. 11] This figure depicts the results of the pathological change observed by HE staining, which was carried out by transplanting skin samples of Darie's disease patients to nude mice. One week after the acceptance of graft, each optimum concentration of dihomo-y-linolenoyldopamine, WIN55,212-2, DPTHF, or DMSO (control) was applied every day for 5 days.

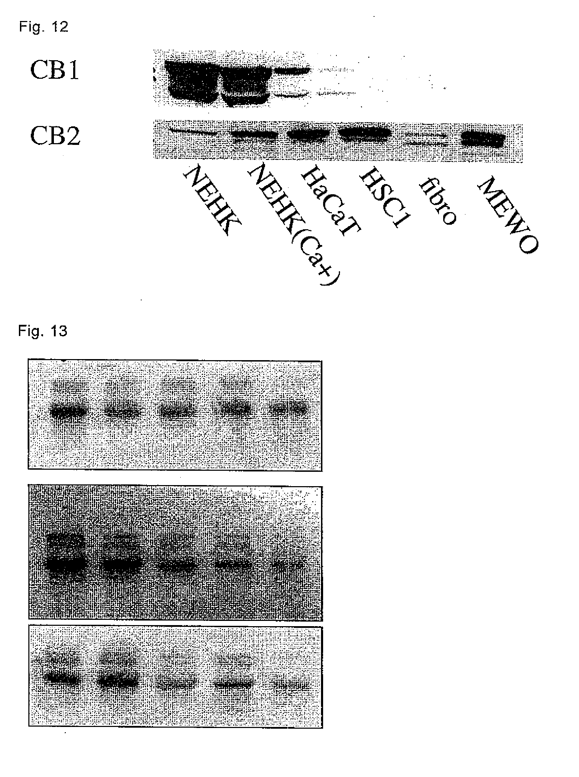

[Fig. 12] This figure depicts the results of analysis of expression of CB1 and CB2 proteins by Western blotting using antibodies specific for CB1 and CB2 receptors as cannabinoid receptors, respectively, in normal cultured epidermal keratinocytes (NHEK), NHEK cells cultured at a high calcium concentration, HaCaT cells and HSC-1 cells each being human epidermal keratinocyte cell lines, human fibroblasts (fibro), and melanocyte cell line (MEWO). In the NHEK cells, HaCaT cells, and HSC-1 cells, the CB1 and CB2 receptor proteins were expressed.
[Fig. 13] This figure depicts the results of the expression of ATP2A2 mRNA observed by Northern blotting, which was carried out by treating epidermal keratinocytes established from skin samples of pathological lesions of Darier's disease patients with WIN55,212-2, dihomo-γ-linolenoyldopamine, or eicosa-11Z,14Z-dienoyldopamine, and then extracting RNA. The results obtained by WIN55,212-2 are shown in the upper row, the results by dihomo-y-linolenoyldopamine are shown in the middle row, and the results by eicosa-11Z,14Z-dienoyldopamine are shown in the lower row. The lanes indicate results at drug concentrations of 10⁻⁶ mol, 10⁻⁷ mol, 10⁻⁸ mol, 10⁻⁹ mol, and 0 mol (control) from the left.

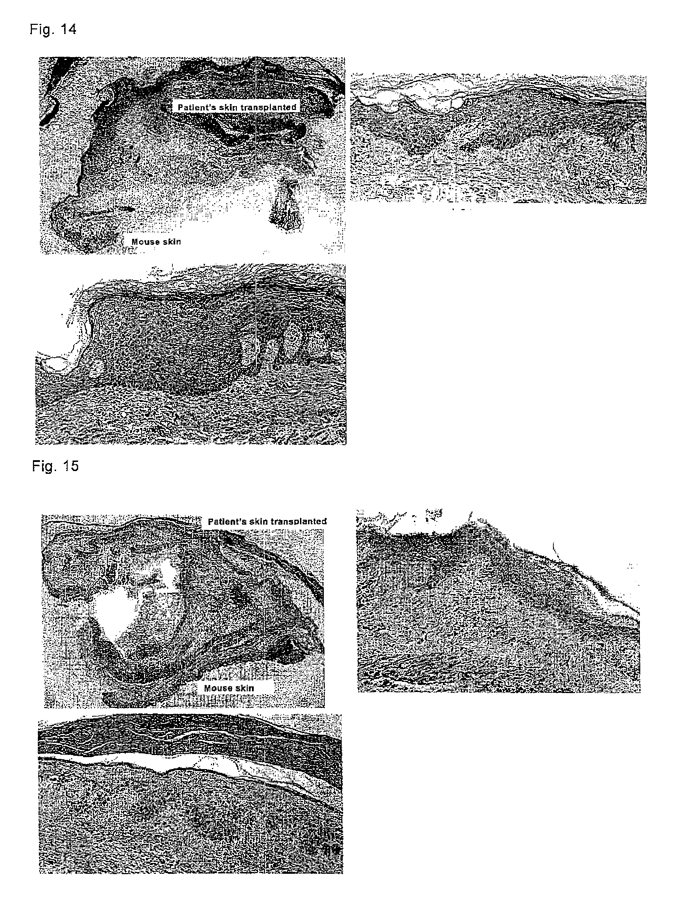

[Fig. 14] This figure depicts pathological changes obtained by transplanting skins of Darie's disease patients to nude mice. After acceptance of graft, WIN55,212-2 was applied for 9 days.
[Fig. 15] This figure depicts pathological changes obtained by transplanting skins of Darie's disease patients to nude mice. After acceptance of graft, dihomo-γ-linolenoyldopamine was applied for 9 days.

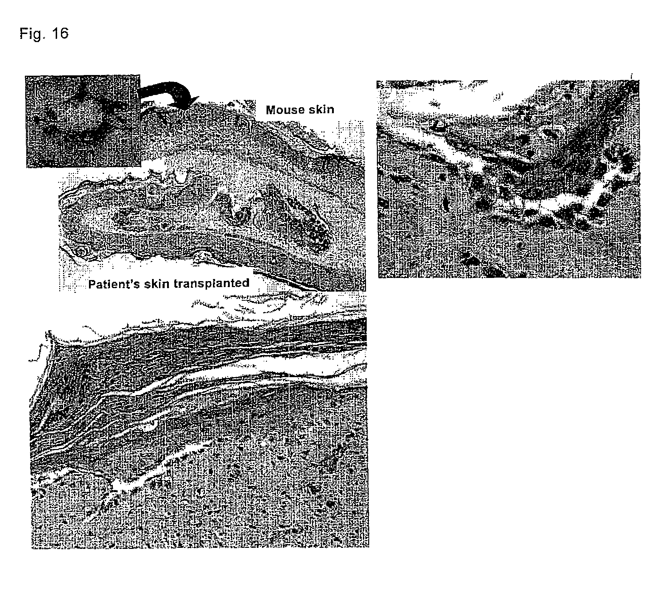

[Fig. 16] This figure depicts pathological changes obtained by transplanting skins of Darie's disease patients to nude mice. After acceptance of graft, a solvent (DMSO) was applied.

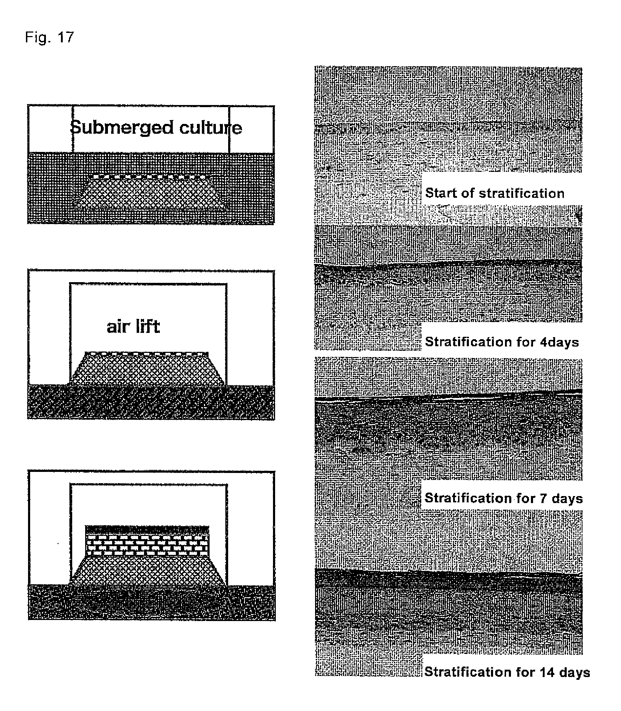

[Fig. 17] This figure depicts a model representing a normal control, indicating a method for three-dimensional culture of a combination of cultured epidermal keratinocytes and fibroblasts or interstitial components of healthy persons, and showing changes of stratification over period of time.

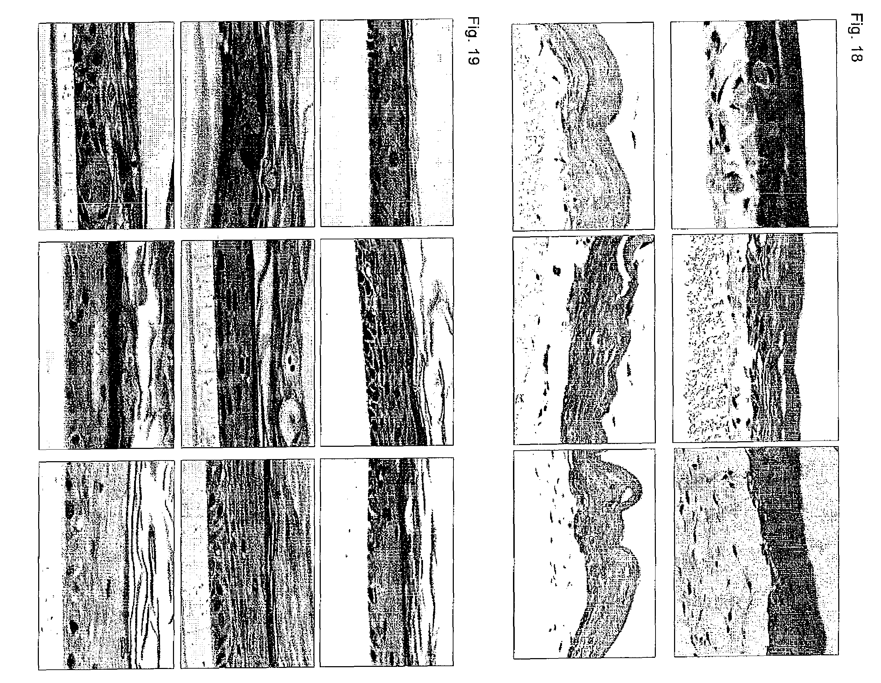

[Fig. 18] This figure depicts actions of medicaments observed in a three-dimensionally cultured skin model utilizing a combination of cultured epidermal keratinocytes and fibroblasts derived from Darie's disease patients. The results obtained after 5 days are shown in the upper row, and the results after 10 days are shown in the lower row. The results obtained by control (DMSO) are shown on the left, the results by WIN55,212-2 are shown in the center, and the results by dihomo-γ-linolenoyldopamine are shown on the right.
[Fig. 19] This figure depicts actions of medicaments observed in a three-dimensionally cultured skin model utilizing a combination of cultured epidermal keratinocytes of Darie's disease patients and interstitial components. The results obtained after 5 days are shown in the upper row, the results after 8 days are shown in the middle row, and the results after 11 days are shown in the lower row. The results obtained by the control (DMSO) are shown on the left, the results by WIN55,212-2 are shown in the center, and the results by dihomo-γ-linolenoyldopamine are shown on the right.

### Best Mode for Carrying out the Invention

The medicament of the present invention is for prophylactic and/or therapeutic treatment of a dermatosis resulting from abnormal calcium metabolism in keratinocytes, and is characterized to contain a substance selected from the group consisting of a cannabinoid agonist and a vanilloid agonist as an active ingredient.

As the cannabinoid agonist, those acting as an agonist for either or both of CB1 and CB2 receptors, expressed by epidermal keratinocytes, can be used.
Examples include, for example, indomethacin morphonylamide, N-acyl derivatives of aminoalcohols such as palmidrol disclosed in Japanese Patent Unexamined Publication No. 5-345722, 2-oxoquinoline derivatives disclosed in Japanese Patent Unexamined Publication No. 2000-256323, 2-imino-1,3-thiazine derivatives disclosed in International Patent Publication WO01/19807, pyridone derivatives disclosed in International Patent Publication WO02/53543, 3,4-dihydroisoquinoline derivatives disclosed in International Patent Publication WO02/10135, pyrazole derivatives disclosed in Japanese Patent Unexamined Publication based on International Patent Application No. 2001-516361, and the like. Palmidrol, indomethacin morphonylamide, or 2-oxoquinoline derivative is preferably used. Examples of non-selective agonists include, for example, anandamide, tetrahydrocannabinoid, and the like. A synthetic cannabinoid agonist, WIN55,212-2 (Drug Discovery Today, Volume 10, Issue 10, pp.693-702, 2005), and the like can be preferably used.

The vanilloid agonist may be an agonist which acts as a selective agonist for any one of six kinds of subtypes or for plural of the receptor, or an agonist which acts as a non-selective agonist for the subtypes of the receptor. Those acting as agonists for one or more kinds of receptors among TRPV1, TRPV3, TRPV4, TRPV5, and TRPV6 expressed on the skin can be preferably used, and those acting as agonists for TRPV3 and/or TRPV4 can be more preferably used. Those acting as selective agonists for TRPV3 and/or TRPV4, and those not activating TRPV1 or having only a weak agonistic action for TRPV1 are most preferred. As the vanilloid agonist, for example, either an endogenous vanilloid agonist or a non-endogenous vanilloid agonist may be used. Examples of the endogenous vanilloid agonist include liposoluble dopamines such as palmitoyldopamine, oleoyldopamine, linoleoyldopamine, γ-linolenoyldopamine, eicosa-11Z,14Z-dienoyldopamine, dihomo-γ-linolenoyldopamine, arachidonoyldopamine, eicosapentaenoyldopamine, docosatetra-7Z,10Z,13Z,16Z-enoyldopamine, docosahexaenoyldopamine, and the like. Among them, linoleoyldopamine, γ-linolenoyldopamine, eicosa-11Z,14Z-dienoyldopamine, dihomo-γ-linolenoyldopamine, and arachidonoyldopamine can be preferably used. Synthetic TRPV agonists such as 2-aminoethoxydiphenyl borate (2-APB) and 4-α-phorbol 12,13-didecanoate (4α-PDD) can also be used. Among them, eicosa-11Z,14Z-dienoyldopamine, dihomo-γ-linolenoyldopamine and 4-α-phorbol 12,13-didecanoate are preferred. However, the agonists are not limited to these examples.

The medicament of the present invention has an action of promoting expression of Serca2 gene, and an action of increasing an expression amount of ATP2A2 protein (calcium pump) encoded by the Serca2 gene to reduce an intracellular calcium concentration. It is known that a calcium ion concentration in keratinocytes significantly affects keratinization process, and keratinization is abnormally promoted by an increase of the calcium ion concentration in keratinocytes to induce a hyperkeratosis condition and to trigger the onset of various diseases. The medicament of the present invention decreases the calcium ion concentration in keratinocytes, thereby abnormally advanced excess keratinization can be controlled to advance normal keratinization. Therefore, the medicament of the present invention has effectiveness for a dermatosis resulting from excessively advanced keratinization, for example, inflammatory keratosis (non-hereditary) and congenital keratoderma (hereditary). The medicament of the present invention can be applied to, for example, Darie's disease and Hailey-Hailey disease, which are congenital dermatoses directly resulting from abnormal calcium metabolism in epidermal keratinocytes, as well as dermatoses resulting from excessively advanced keratinization other than Darie's disease and Hailey-Hailey disease, including congenital keratoderma, psoriasis vulgaris, and the like.

In particular, it is known that, in Darie's disease, an expression amount of ATP2A2 protein is reduced to about half of that observed in healthy persons. By the administration of the medicament of the present invention to a Darie's disease patient, an expression amount of ATP2A2 protein from the basal layer to the spinous layer of keratinocytes can be increased, and as a result, the onset of Darie's disease can be prevented, or treatment of Darie's disease for improvement, amelioration or the like of the symptoms can be performed. Although the medicament of the present invention has high effectiveness especially on Darie's disease, said medicament also has effectiveness on abnormal skin keratinization diseases induced by abnormal calcium metabolism in the keratinocytes due to abnormal expression of the calcium pump in the keratinocytes, for example, Hailey-Hailey disease and the like.

The medicament of the present invention is prepared as a medicament containing a substance selected from the group consisting of the aforementioned cannabinoid agonists and vanilloid agonists. As the active ingredient, a physiologically acceptable salt, or a hydrate, a solvate or the like of the substance selected from the group consisting of a cannabinoid agonist and a vanilloid agonist may also be used. Two or more kinds of the aforementioned active ingredients may be used in combination for the medicament of the present invention. The medicament of the present invention may be preferably administered as a pharmaceutical composition for oral or parenteral administration, which can be prepared by a method well known to those skilled in the art. Examples of pharmaceutical composition suitable for oral administration include, for example, tablets, capsules, powders, subtilized granules, granules, solutions, syrups, and the like, and examples of pharmaceutical composition suitable for parenteral administration include, for example, injections, suppositories, inhalants, ointments, creams, solutions, patches, and the like. Type of the pharmaceutical composition can be suitably chosen according to type of active ingredient. The medicament of the present invention is intended to be applied to a dermatosis, it is also preferable to prepare the medicament as a preparation for external application on skin such as ointment, cream, solution, and patch.

The aforementioned pharmaceutical composition can be prepared by adding pharmacologically and pharmaceutically acceptable additives. Example of the pharmacologically and pharmaceutically acceptable additives include, for example, excipients, disintegrating agents or disintegrating aids, binders, lubricants, coating agents, dyes, diluents, bases, dissolving agents or dissolving aids, isotonic agents, pH modifiers, stabilizers, propellants, tackifiers, and the like.

Dose of the medicament of the present invention is not particularly limited, and the dose can be suitably increased or reduced depending on various factors to be usually taken into consideration, such as type of an active ingredient, body weight and age of a patient, symptoms, and a route of administration. In the case of oral administration, the medicament can generally be used in the range of about 0.01 to 1,000 mg per day for adults, which dose can be suitably increased or reduced. Period for application of the medicament of the present invention is not particularly limited. When the medicament is applied to Darie's disease, application can be started prior to summer season when eruptions is aggravated, or in adolescent period during when Darie's disease develops, thereby the onset thereof or advance of the symptoms is suppressed and aggravation to severe eruptions can be avoided to be kept in mild symptoms.

### Examples

The present invention will be more specifically explained with reference to examples. However, the scope of the present invention is not limited by the following examples.

### Example 1

Normal human epidermal keratinocytes (NHEK) were cultured in a serum-free medium (KGM medium) from which only hydrocortisone was excluded. A test substance dissolved in dimethyl sulfoxide (DMSO) as a solvent was added to the medium and culture was continued, and amount of mRNA of Serca2 gene in the epidermal keratinocytes was quantified by Northern blotting after 3, 6, and 9 hours. It was observed that induction of Serca2 gene was induced by several kinds of synthetic cannabinoid agonists including WIN55,212-2, methanandamide, BML-190 (FEBS Lett., 536, pp.157-160, 2003) and the like among several hundreds kinds of test substances. RNA was extracted 0, 3, 6, 12, 18, 24, and 48 hours after the addition of WiN55,212-2 to the epidermal keratinocytes, and expression of Serca2 gene was observed by Northern blotting. The results are shown in Fig. 1. Further, various concentrations of WiN55,212-2 (10⁻⁵, 10⁻⁶, 10⁻⁷,3 x 10⁻⁸, 10⁻⁸, 3 x 10⁻⁹, 10⁻⁹ mol) or DMSO as a control was added to epidermal keratinocytes, and then RNA was extracted after 6 hours, and expression of SERCA2 gene was observed by Northern blotting. The results are shown in Fig. 2.

### Example 2

Test substances including various kinds of vanilloid agonists were examined in the same manner as that of Example 1. It was confirmed that oleoyldopamine, linoleoyldopamine, γ-linolenoyldopamine, eicosa-11Z,14Z-dienoyldopamine, dihomo-γ-linolenoyldopamine, arachidonoyldopamine, eicosapentaenoyldopamine, docosatetra-7Z,10Z,13Z,16Z-enoyldopamine, and docosahexaenoyldopamine, which are endogenous vanilloid agonists, as well as 2-aminoethoxydiphenyl borate (2-APB) and 4-α-phorbol 12,13-didecanoate (4α-PDD), which are synthetic TRPV agonists, induced the Serca2 gene. Among them, linoleoyldopamine, γ-linolenoyldopamine, eicosa-11Z,14Z-dienoyldopamine, dihomo-γ-linolenoyldopamine, and arachidonoyldopamine had potent inducing action.

The above Serca2 mRNA inducing action was observed 3 hours after the addition of each test substance, and reached to the maximum 6 to 9 hours after the addition. The vanilloid agonists induced the Serca2 gene expression at a lower concentration compared with the cannabinoid agonists used in Example 1. The effective concentrations of the vanilloid agonists are substantially consistent with the physiological concentrations thereof in cells. Therefore, it is considered that the medicament of the present invention acts on the vanilloid receptors expressed in the epidermal keratinocytes. When DPTHF (diphenyltetrahydrofuran, J. Biol. Chem., 280, pp.15928-15941, 2005) as a vanilloid antagonist was coexisted, the inducing action of the vanilloid agonists on the Serca2 gene expression disappeared (Fig. 7). It was also observed that the Serca2 gene expression was similarly enhanced by vanilloid agonists in HaCaT cells, which is a typical human epidermal keratinocyte cell line.

### Example 3

Human skin under a state of organ culture was added with optimum concentration of dihomo-γ-linolenoyldopamine or DMSO (control), and after 24 hours later, the skin was fixed, and then localization of ATP2A protein, which is the causative protein of Darie's disease, was identified by immunostaining. In the epidermal section added with dihomo-γ-linolenoyldopamine, excessively advanced expression in the basal layer was observed, indicating localization of the ATP2A protein (Fig. 8).

### Example 4

Skins of healthy persons were transplanted to nude mice, and after one week under acceptance of graft, optimum concentrations of dihomo-γ-linolenoyldopamine, WIN55,212-2, DPTHF as a vanilloid antagonist, and DMSO (control) were each applied every day for 5 days. The skin samples where the application was made were fixed after biopsy, and localization of ATP2A protein was identified by immunostaining. The results are shown in Fig. 9. In the epidermal sections added with dihomo-γ-linolenoyldopamine or WIN55,212-2, excessively advanced expression in the basal layer was observed, indicating localization of ATP2A protein. Whilst, with the application of DPTHF, decrease of expression of ATP2A2 was observed.

### Example 5

Optimum concentrations of dihomo-γ-linolenoyldopamine, WIN55,212-2, DPTHF as a vanilloid antagonist, and DMSO (control) were each added to skin samples of healthy persons and kept under a state of organ culture in the same manner as in Example 3. The skin samples were fixed after 24 to 48 hours, and pathological changes were observed by HE staining. The results are shown in Fig. 10. The epidermal sections added with dihomo-γ-linolenoyldopamine or WIN55,212-2 remained in a state of physiological skin not distinguishable from healthy skins, in the same manner as the section added with DMSO as the control. Whilst, when DPTHF as a vanilloid antagonist was added, pathological images similar to those of Darie's disease such as individual keratinization and acantholysis were observed.

### Example 6

Skin samples of Darie's disease patients were collected under informed consent of the patients, and transplanted to nude mice, and after one week under acceptance of graft, optimum concentrations of dihomo-γ-linolenoyldopamine, WIN55,212-2, and DMSO (control) were each applied to the skin samples every day for 5 days. The skins where the application was made were fixed after biopsy, and pathological changes were observed by HE staining. The results are shown in Fig. 11. When DMSO as the control was applied, pathological changes characteristic to Darie's disease were remained, for example, acantholysis and dyskeratosis were observed in the basal layer, and absence of formation of granular layer was observed. Whilst, when dihomo-γ-linolenoyldopamine or WIN55,212-2 was applied, these pathological changes characteristic to Darie's disease disappeared.

### Example 7

Epidermal keratinocytes were collected from skins of pathological lesions of 3 Darier's disease patients, and cultured to establish cell lines. The cells were treated with WIN55,212-2 or a vanilloid agonist (dihomo-γ-linolenoyldopamine or eicosa-11Z,14Z-dienoyldopamine), and after 6 hours, RNAs were extracted and the expression of ATP2A2 mRNA was observed by Northern blotting. As a result, concentration-dependent promotion of expression of ATP2A2 was observed with the cannabinoid agonist and the vanilloid agonist. A typical result among the three patients is shown in Fig. 13. When the epidermal keratinocytes of the other two Darie's disease patients were used, similar results were obtained.

### Example 8

Skin samples of Darie's disease patients with an area of 1 to 2 cm square were transplanted to nude mice, and acceptance of graft was confirmed after 7 days. Thereafter WIN55,212-2 or dihomo-γ-linolenoyldopamine was applied for 5 to 9 days using dimethyl sulfoxide (DMSO) as a solvent, and effectiveness of the drugs was determined on the basis of pathological examinations. This method is effective for successful determination of efficacy of a drug under a condition closest to the affected patient's skin. When WIN55,212-2 as a cannabinoid agonist was applied for 9 days, dyskeratosis, parakeratosis, and blistering were disappeared, and normal keratinization was observed with recovered granular layer, i.e., the pathological pictures of Darie's disease were observed to be ameliorated to those of healthy skin. Further, when dihomo-γ-linolenoyldopamine as a vanilloid agonist was applied for 9 days, dyskeratosis and blistering disappeared, and the granular layer also slightly recovered, but keratinization accompanied by parakeratosis slightly remained. Where a solvent (DMSO) was applied as the control, dyskeratosis, parakeratosis, and blistering remained, the granular layer remained disappeared, and pathological pictures of Darie's disease were observed even after the transplantation. These results are shown in Figs. 14 to 16. It is considered that the medicament of the present invention has sufficient effectiveness on inflammatory keratosis such as psoriasis vulgaris, as well as on Darie's disease, because potent therapeutic effect that achieves successful recover of even the formation of normal granular layer was observed when the cannabinoid agonist was applied.

### Example 9

In order to construct a model skin of Darie's disease which is readily reproducible and can be repetitively applied for assays, a Darie's disease model skin was constructed by three-dimensional culture of a combination of cultured epidermal keratinocytes and fibroblasts of Darie's disease patients, or three-dimensional culture of a combination of cultured epidermal keratinocytes Darie's disease patients and interstitial components. WIN55,212-2 or dihomo-γ-linolenoyldopamine was added to the medium of the above model skin to confirm the actions thereof. The results are shown in Figs. 18 and 19. As a result, the pathological pictures of individual keratinization or deviation of skins equivalents (acantholysis) disappeared after the addition of the medicaments, and recovery of three-dimensional skin having quality similar to that of healthy skin was observed. With the DMSO treatment as a control, individual keratinization and acantholysis remained, and pathological conditions corresponding to the pathological pictures of Darie's disease were observed. These results indicate that the medicament of the present invention exhibits sufficient effectiveness not only on Darie's disease but also on inflammatory keratosis such as psoriasis vulgaris.

### Industrial Applicability

The medicament of the present invention is useful for prophylactic and/or therapeutic treatment of a dermatosis resulting from excessively advanced keratinization, for example, non-hereditary inflammatory keratosis and hereditary congenital keratosis.

## Claims

1. A medicament for prophylactic and/or therapeutic treatment of a dermatosis resulting from excessively advanced keratinization, which comprises a substance selected from the group consisting of a cannabinoid agonist and a vanilloid agonist as an active ingredient.

2. The medicament according to claim 1, wherein the dermatosis resulting from excessively advanced keratinization is inflammatory keratosis (non-hereditary) or congenital keratoderma (hereditary).

3. The medicament according to claim 1, wherein the dermatosis resulting from excessively advanced keratinization is a congenital dermatosis resulting from abnormal calcium metabolism in epidermal keratinocytes.

4. The medicament according to claim 3, wherein the congenital dermatosis resulting from abnormal calcium metabolism in epidermal keratinocytes is Darie's disease or Hailey-Hailey disease.

5. The medicament according to any one of claims 1 to 4, which comprises a vanilloid agonist as an active ingredient.

6. The medicament according to claim 5, wherein the vanilloid agonist is eicosa-11Z,14Z-dienoyldopamine, dihomo-γ-linolenoyldopamine or 4-α-phorbol 12,13-didecanoate.
